# EUROPEAN PATENT SPECIFICATION

(11) **EP 3 548 116 B1**
(45) Date of publication and mention of the grant of the patent: **27.04.2022**
(21) Application number: 17803940.0
(22) Date of filing: 23.11.2017
(51) Int. Cl.: A61M 5/20, A61M 5/32, A61M 5/315

(54) **ACTIVATION ASSEMBLY FOR A MEDICAMENT DELIVERY DEVICE**
AKTIVIERUNGSANORDNUNG FÜR EINE MEDIKAMENTENABGABEVORRICHTUNG
ENSEMBLE D'ACTIVATION POUR DISPOSITIF D'ADMINISTRATION DE MÉDICAMENTS

(30) Priority: 29.11.2016 WO PCT/EP2016/201284
(43) Date of publication of application: 09.10.2019
(73) Proprietor: SHL Medical AG, 6302 Zug (CH)
(72) Inventor: HOLMQVIST, Anders, 139 40 Värmdö (SE)
(86) International application number: PCT/EP2017/080189
(87) International publication number: WO 2018/099795

(56) References cited:
- WO-A1-2016/055305
- WO-A1-2016/120207
- WO-A1-2016/128207
- WO-A2-2016/034407

## Description

### TECHNICAL FIELD

The present disclosure generally relates to medicament delivery devices. In particular, it relates to an activation assembly for a medicament delivery device.

### BACKGROUND

Modern medicament delivery devices, such as auto-injectors, are designed to facilitate medicament administration in a manner which allows users to administer medicaments themselves. This freedom for patients to handle medicament delivery has lead to the concept of adherence, or compliance, to become an increasingly important area in treatment of illnesses. Adherence involves monitoring of a patient's medication administration scheme as prescribed by a physician and evaluation of whether a prescribed medicament and medicament administration scheme has been successful or not in treating the illness of the patient.

It has been found that it is relatively common that a user does not administer the medication as prescribed. Reasons for a patient's failure to comply with the prescribed scheme include forgetfulness, pain associated with drug administration or discomfort experienced from side effects of the medication.

Failure to comply with a drug administration scheme may result in that the patient can experience poor recovery from an illness, and it may furthermore result in secondary diseases requiring additional medical attention. This may in turn bring unnecessary pressure on the healthcare system.

In view of the above, it is in the interest of both patients' and the healthcare to facilitate monitoring to increase the occurrences of medicament administration as prescribed.

WO2007/107564 A1 discloses an electronic module for mechanical medication delivery devices, and aims at monitoring the operation of a medication delivery device. This document discloses an electronic module that is attached onto a medication delivery device. The electronic module is capable of measuring acoustical and/or vibrational signals generated in response to relative movements of internal parts of the medication delivery device to which the electronic module is attached. Such internal parts can be mechanical parts which during movement generate for example acoustical sounds, such as click sounds. The electronic module is powered by a built-in battery which powers the module when for example a capacitive touch pad is activated. This activation is performed when for example a finger tip is positioned on the touch pad.

In WO2007/107564 A1 the battery and thus the electronic module is activated provided a user touches the touch pad located on the electronic module. This touch pad is not associated with the mechanical operation of the medication delivery device. There is hence a risk that a user will forget to activate the electronic module prior to drug administration. This would result in that the following drug administration would not be registered. There are also some monitoring devices in the market such as those disclosed in WO 2016/128207A1, WO2016/055305A1, WO2016/120207A1, WO2016/034407A2 and WO2007/107564A1.

### SUMMARY

An object of the present disclosure is to provide an activation assembly for a medicament delivery device which solves or at least mitigates problems of the prior art.

There is hence according to a first aspect of the present disclosure provided a medicament delivery device comprising: a housing and an activation assembly for a medicament delivery device, wherein the activation assembly comprises: a delivery member cover provided with legs and configured to be biased in a proximal direction, a movable sleeve configured to be received between the legs, and an activation sleeve configured to be biased in the proximal direction, wherein the delivery member cover is configured to be moved in an axial direction between a first position relative to the movable sleeve and a second position relative to the movable sleeve, wherein movement of the delivery member cover from the first position to the second position causes movement of the movable sleeve from a first movable sleeve position to a second movable sleeve position, wherein the delivery member cover is configured to axially displace the activation sleeve in a distal direction when the delivery member cover is moved towards the second position, the activation sleeve being configured to thereby provide a first actuation of a sensor of an electronics assembly of the medicament delivery device, wherein the activation sleeve is configured to move in the proximal direction when the delivery member cover returns from the second position to the first position, thereby providing a second actuation of the sensor, and wherein the movable sleeve is configured to prevent the delivery member cover from moving from the first position to the second position when the delivery member cover has returned from the second position to the first position.

The activation sleeve is hence configured, upon distal movement of the delivery member cover, to provide a first actuation of a sensor of an electronics assembly of a medicament delivery device. This actuation of the sensor may for example provide a registration of the commencement of a medicament administration by the electronics assembly. It furthermore ensures that the sensor is actuated whenever the delivery member cover is moved distally, which is during a medicament administration procedure, so that compared to WO2007/107564 A1, it may be made certain that registration of a medicament administration can always be obtained. Additionally, the subsequent proximal movement of the delivery member cover and thus of the activation sleeve causes the second actuation of the sensor, whereby it may be registered by the electronics assembly that the medicament delivery procedure is being completed.

The activation sleeve may be configured to constantly contact the sensor while the delivery member cover is in the second position, so that the second actuation of the sensor occurs when the activation sleeve is released from or ceases being in contact with the sensor.

The movable sleeve is rotatable relative to the delivery member cover, and movement of the delivery member cover from the first position to the second position causes rotation of the movable sleeve, wherein the first movable sleeve position is a first rotational position and the second movable sleeve position is a second rotational position.

The delivery member cover has a distal end surface configured to cooperate with a radially extending portion of the movable sleeve, forming a cam surface, wherein cooperation between the distal end surface and the radially extending surface causes the rotation of the movable sleeve.

The activation sleeve has proximally extending arms configured to be axially aligned with and to cooperate with a respective leg of the delivery member cover, the delivery member cover thereby pushing the activation sleeve when moved from the first position to the second position causing the axial displacement of the activation sleeve.

The activation sleeve has a distally extending arm configured to actuate the sensor.

According to one embodiment the movable sleeve has a tangentially extending radially flexible first tongue configured to allow the delivery member cover to move over the first tongue towards the second position when the movable sleeve is in the first rotational position and to prevent the delivery member cover to move from the first position to the second position when the movable sleeve is in the second rotational position and the delivery member cover has returned to the first position.

According to one embodiment the first tongue has an increasing thickness in the tangential direction, whereby the first tongue is configured to enable the delivery member cover to move over the first tongue, in the axial direction, from the first position towards the second position, when the movable sleeve is in the first rotational position.

According to one embodiment the movable sleeve has a radially outwards extending protrusion configured to cooperate with the delivery member cover to prevent rotation of the movable sleeve from the second rotational position to the first rotational position.

According to one embodiment the movable sleeve has a tangentially extending radially flexible second tongue, wherein the radially outwards extending protrusion is provided on the second tongue.

One embodiment comprises an electronics assembly including an energy storage unit and processing circuitry configured to register activation of a medicament administration procedure.

According to one embodiment the electronics assembly comprises a sensor in the form of a switch, and the activation sleeve is configured to provide the first actuation of the switch.

According to one embodiment the switch extends radially outwards and is radially flexible, wherein the activation sleeve is configured to provide the first actuation of the switch by moving distally, over the switch, thereby pressing the switch radially inwards and to provide the second actuation of the switch by moving proximally, away from the switch, thereby releasing the switch from being pressed radially inwards.

According to one embodiment the movable sleeve is configured to be arranged proximally relative to the activation sleeve.

There is according to a second aspect of the present disclosure provided a medicament delivery device comprising: a housing, and an activation assembly according to the first aspect, configured to be received by the housing.

The medicament delivery device also includes the electronics assembly, either arranged inside the housing, or included in a supplementary device, i.e. an add-on device, configured to be mounted to the main body of the medicament delivery device.

One embodiment comprises a plunger rod configured to move axially inside the housing from an initial position to an end position, and an end click member configured to be released and thrown in the distal direction when the plunger rod has travelled a majority of the distance from the initial position to the end position, and an end click sensor configured to detect impact of the end click member upon being released, causing the electronics assembly to register completion of medicament expulsion.

In this manner, three different actuations/detections may be registered by the electronics assembly, namely the first and second actuation of the sensor, and also the detection of impact of the end click member. It may thereby be possible to determine, by means of the electronics assembly, commencement of medicament administration based on the first actuation, completion of, or almost completion of, medicament administration by means of detecting impact of the end click member, and determining that e.g. the delivery member cover has been maintained in the second position for e.g. a predetermined amount of time post administration completion, to ensure correct administration, based on for example the lapse of a certain time between the detection of the impact of the end click member and the second actuation.

Generally, all terms used in the claims are to be interpreted according to their ordinary meaning in the technical field, unless explicitly defined otherwise herein. All references to "a/an/the element, apparatus, component, means, etc. are to be interpreted openly as referring to at least one instance of the element, apparatus, component, means, etc., unless explicitly stated otherwise.

### BRIEF DESCRIPTION OF THE DRAWINGS

The specific embodiments of the inventive concept will now be described, by way of example, with reference to the accompanying drawings, in which:
Fig. 1 is a perspective view of an example of a medicament delivery device;
Fig. 2 is an exploded view of the medicament delivery device in Fig. 1;
Fig. 3 is a perspective view of an example of an activation assembly, for use in the medicament delivery device in Fig. 1;
Fig. 4 is a perspective view of certain components of the activation assembly in Fig. 3;
Fig. 5 shows a side view, with inter alia the housing removed, of a distal end of the medicament delivery device in Fig. 1;
Fig. 6 is a side view, with the housing removed, of a first state of operation of the medicament delivery device;
Figs 7a-b show the medicament delivery device, with the housing removed, when a delivery member cover has been moved distally to cooperate with an activation sleeve;
Figs 8a-b show the medicament delivery device, with the housing removed, when a delivery member cover has been moved fully distally;
Figs 9a-b show the medicament delivery device, with the housing removed, when a delivery member cover has returned to its first position, its initial position; and
Fig. 9c shows an example of a blocking feature for preventing distal movement of the delivery member cover once returned to the initial position shown in Figs 9a-b.

### DETAILED DESCRIPTION

The inventive concept will now be described more fully hereinafter with reference to the accompanying drawings, in which exemplifying embodiments are shown. The inventive concept may, however, be embodied in many different forms and should not be construed as limited to the embodiments set forth herein; rather, these embodiments are provided by way of example so that this disclosure will be thorough and complete, and will fully convey the scope of the inventive concept to those skilled in the art. Like numbers refer to like elements throughout the description.

With the term "proximal end" of an activation assembly is meant that end which is to be pointed towards the injection site during medicament injection. The same considerations also apply when referring to any component of the activation assembly. The "distal end" is the opposite end relative to the proximal end. With "proximal direction" and, equivalently, "proximally" is meant a direction from the distal end towards the proximal end, along the central axis of the activation assembly. With "distal direction" or "distally" is meant the opposite direction to "proximal direction".

The present disclosure relates to an activation assembly for a medicament delivery device. The medicament delivery device may for example an auto-injector or an eye dispenser.

The activation assembly comprises a proximally biased delivery member cover, a movable sleeve and a proximally biased activation sleeve. The delivery member cover has legs extending in the distal direction, and the delivery member cover is configured to receive the movable sleeve between the legs.

The delivery member cover is configured to be displaced axially relative to the movable sleeve, between a first position and a second position. The delivery member cover is configured to cooperate with the movable member so that when the delivery member cover is moved from the first position towards the second position, the linear movement of the delivery member cover causes movement of the movable sleeve, from a first movable sleeve position to a second movable sleeve position. In a typical example, the linear movement of the delivery member cover causes rotational movement of the movable sleeve, and the first movable sleeve position and the second movable sleeve position is a first rotational position and a second rotational position, respectively. The movable sleeve could according to one example also move a distance axially while being rotated by the linear movement of the delivery member cover.

The delivery member cover is furthermore configured to axially displace the activation sleeve when moved distally towards the second position. While being moved distally, the activation sleeve is configured to provide a first actuation of a sensor of an electronics assembly of a medicament delivery device. When the delivery member cover is returned from the second position to its initial position, i.e. the first position, the proximally biased activation sleeve will also move in the proximal direction, away from the sensor, to thereby provide a second actuation of the sensor.

The movable sleeve is furthermore configured to prevent movement of the delivery member cover from the first position towards the second position once the delivery member cover has returned from the second position to the first position.

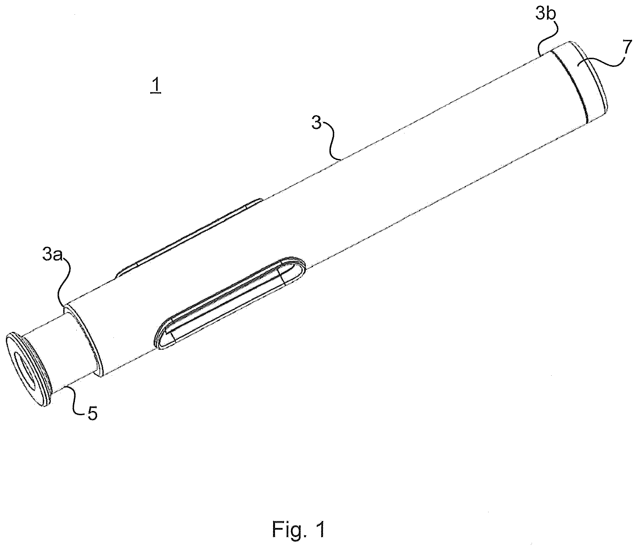

An example of an activation assembly as well as of a medicament delivery device comprising the activation assembly will now be described with reference to Figs. 1-9c.

Fig. 1 shows an example of a medicament delivery device. The particular example shows an auto-injector, but the medicament delivery device could alternatively be for example an eye dispenser.

The exemplified medicament delivery device 1 has a housing 3, having a proximal end 3a, and a distal end 3b, a delivery member cover 5 configured to be received by the housing 3, and an end cap 7. The delivery member cover 5 is configured to be axially displaced relative to the housing 3, between an extended position shown in Fig. 1, and a retracted position, in which the delivery member cover 5 is further received by the housing 3. The delivery member cover 5 is configured to be rotationally locked relative to the housing 3. The delivery member cover 5 is thus only able to move axially relative to the housing 3.

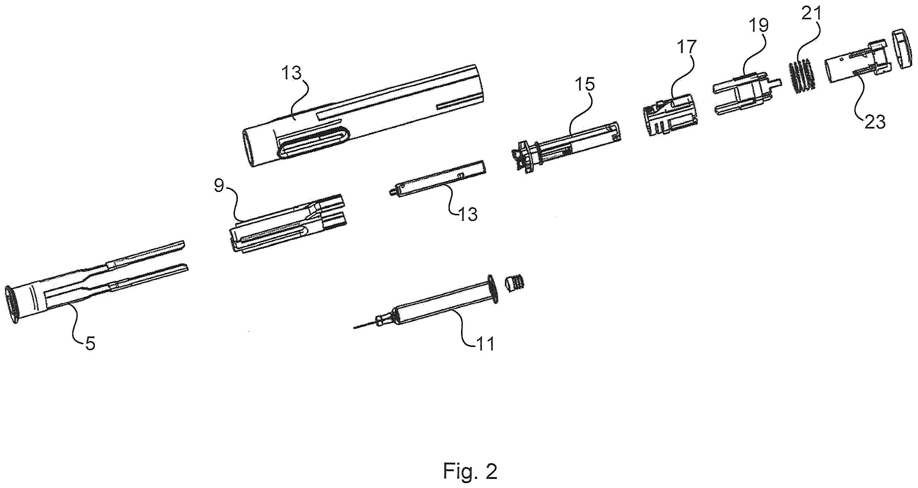

Fig. 2 shows an exploded view of the medicament delivery device 1, showing a number of internal components thereof. Hereto, the medicament delivery device 1 further comprises a medicament container holder 9 configured to hold a medicament container 11, a plunger rod 13, a tubular extension member 15 configured to receive the plunger rod 13 in an axial opening, a movable sleeve 17, an activation sleeve 19, a resilient member 21, for example a spring, configured to bias the activation sleeve 19 in the proximal direction, and a tubular end member 23.

The medicament delivery device 1 may further comprise an electronics assembly, configured to be mounted for example under the end cap 7, i.e. proximally arranged relative to the end cap 7. Alternatively, the medicament delivery device 1 could be configured to be fitted with a supplementary device comprising the electronics assembly.

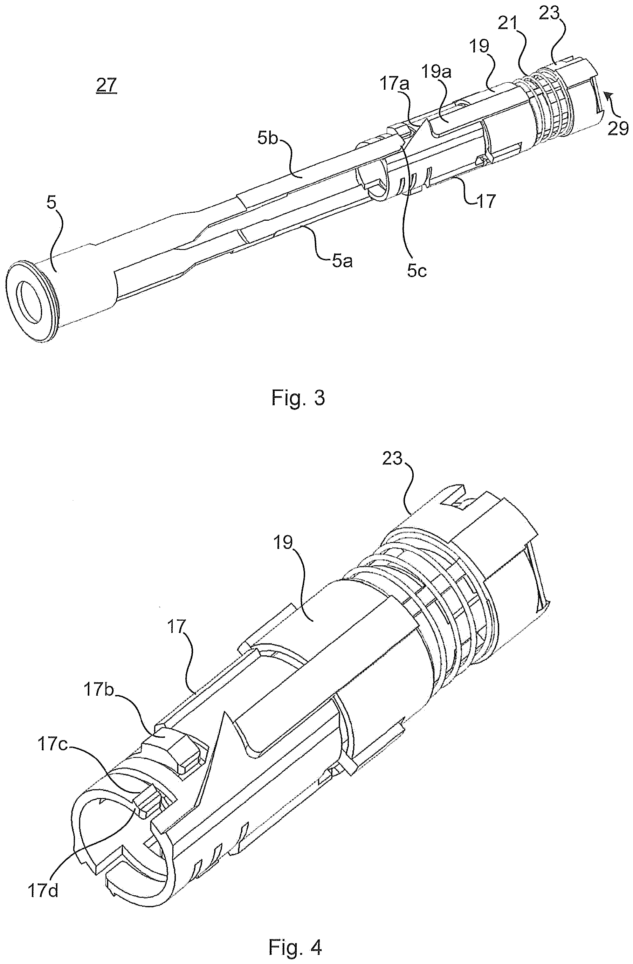

Fig. 3 shows a perspective view of an activation assembly 27 of the medicament delivery device 1, comprising the delivery member cover 5, the movable sleeve 17 and the activation sleeve 19. In the present example, the movable sleeve 17 is a rotator. The activation assembly 27 may also include an electronics assembly 29 arranged at a distal end portion of the activation assembly 27.

The delivery member cover 5, which has a tubular proximal end portion, has a first leg 5a and a second leg 5b extending in the distal direction. The movable sleeve 17 is configured to be received between the first leg 5a and the second leg 5b. The delivery member cover 5 has a distal end surfaces 5c, defining the respective end of the first leg 5a and the second leg 5b. The movable sleeve 17 has a radially extending portion 17a, forming a cam surface, configured to cooperate with a distal end surface 5c of a leg 5a, 5b. The movable sleeve 17 may have two such radially extending portions 17a, each configured to cooperate with a respective distal end surface 5c.

As previously mentioned, the delivery member cover 5 is configured to be moved axially between an extended position and a retracted position relative to the housing 3. The delivery member cover 5 is also configured to move axially relative to the movable sleeve 17, between a first position relative to the movable sleeve 17, corresponding to the extended position relative to the housing 3, and a second position relative to the movable sleeve 17, corresponding to the retracted position relative to the housing 3. In Fig. 3, the delivery member cover 5 is in the first position relative to the movable sleeve 17.

When the delivery member cover 5 is moved from the first position towards the second position relative to the movable sleeve 17, the distal end surface 5c cooperates with the radially extending portion 17a of the movable sleeve 17. Linear movement of the delivery member cover 5, from the first position towards the second position thereby causes movement of the movable sleeve 17, from a first movable sleeve positon to a second movable sleeve position. In particular, the linear movement of the delivery member cover 5 causes rotation of the movable sleeve 17 as the distal end surface 5c slides along the radially extending portion 17a, the cam surface, of the movable sleeve 17 and the delivery member cover 5 is moved proximally. The movable sleeve 17 is then rotated from the first movable sleeve position, which according to the present example is a first rotational position, to the second movable sleeve position, which is a second rotational position.

The activation sleeve 19 has a main body provided with proximally extending legs 19a, of which one is visible in Fig. 3. The proximally extending legs 19a are configured to be aligned with a respective leg 5a and 5b of the delivery member cover 5. Initially, when the delivery member cover 5 is in the first position relative to the movable sleeve 17, and the movable sleeve is in the first rotational position, the radially extending portion 17a is located in between a leg 5a, 5b of the delivery member cover 5 and a proximally extending leg 19a of the activation sleeve 19, as shown in Fig. 3. When the delivery member cover 5 has been moved a certain distance towards the second position and the movable sleeve 17 has been rotated to a certain degree, the radially extending portion 17a will be rotated away from between the proximally extending leg 19a and leg 5a, and thus the delivery member cover 5 will be set in direct contact with the activation sleeve 19. Further distal movement of the delivery member cover 5 will thereby cause axial displacement of the activation sleeve 19, in the distal direction.

Turning now to Fig. 4, the exemplified movable sleeve 17 has a tangentially extending radially flexible first tongue 17b configured to allow the delivery member cover 5 to move over the first tongue 17b from the first position towards the second position, when the movable sleeve 17 has not yet been fully rotated from the first rotational position to the second rotational position.

The first tongue 17b is furthermore configured to prevent the delivery member cover 5 to move from the first position to the second position when the movable sleeve 17 is in the second rotational position and the delivery member cover 5 has returned to the first position.

The first tongue 17b has an increasing thickness in the tangential direction in a tangential direction pointing away from the radially extending portion 17a. The first tongue 17 is thereby configured to enable the delivery member cover 5 to move over it, in the axial direction, when the delivery member cover 5 is moved from the first position towards the second position, and the movable sleeve 17 is being rotated towards the second rotational position.

According to the present example, the movable sleeve 17 has a radially outwards extending protrusion 17c configured to cooperate with the delivery member cover 5 to prevent rotation of the movable sleeve 17 from the second rotational position to the first rotational position. According to the present example, the movable sleeve 17 has a tangentially extending radially flexible second tongue 17d, and the radially outwards extending protrusion 17c is provided on the second tongue 17d.

As an alternative to the configuration with the second tongue 17d, the tubular end member 23, which is configured to be received by a distal portion of the movable member 17, may have an outer surface provided with an engagement structure configured to engage with the movable member 17 when the movable member 17 is set in the second rotational position.

The resilient member 21 may be configured to be arranged between the tubular end member 23 and a distal end of the activation sleeve 19, to thereby bias the activation sleeve 19 proximally.

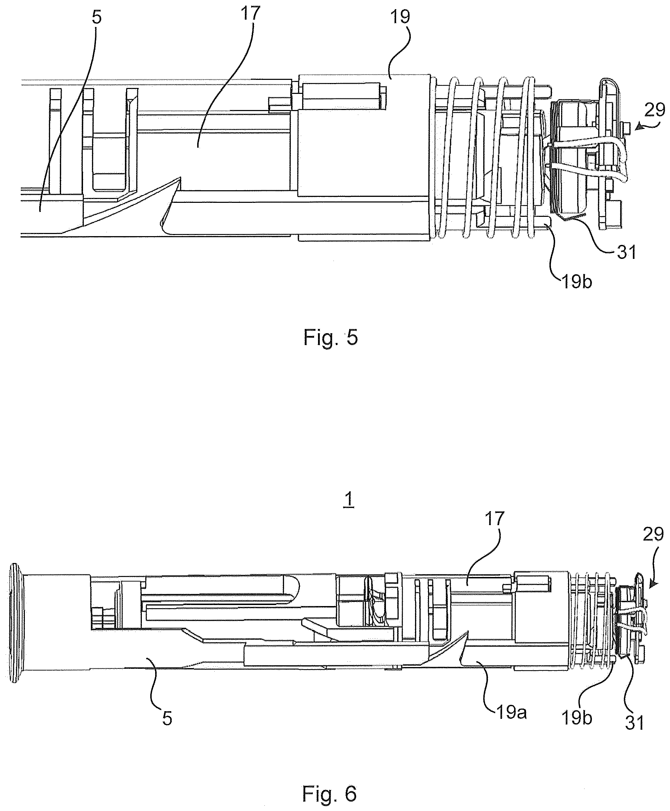

Fig. 5 shows a distal end portion of the medicament delivery device 1, including an electronics assembly 29 arranged distally relative to the activation sleeve 19. The tubular end member 23 has been removed to expose the electronics assembly 29.

The electronics assembly 29 may comprise an energy storage unit, for example a battery, or in case the medicament delivery device 1 is capable of energy harvesting, a capacitor, processing circuitry configured to be powered by the energy storage unit, a storage medium comprising computerexecutable components configured to be run on the processing circuitry, and a wireless transmitter configured to transmit data processed by the processing circuitry. The electronics assembly 29 furthermore comprises a sensor 31, for example a switch. According to the present example, the sensor 31 is a switch which has an elongated axial extension and which extends radially outwards, and which is radially flexible.

The activation sleeve 19 has a distally extending arm 19b configured to actuate the sensor 31. In Fig. 5 the delivery member cover 5 is in the first position and the movable sleeve 17 is in the first rotational position. Hereto, the activation sleeve 19 is in an initial position, in which it has not been moved in the distal direction. The sensor 31, i.e. the switch, is thus in this state of the medicament delivery device 1 not arranged in contact with the distally extending arm 19b. This state is better visible in Fig. 6 which shows the entire medicament delivery device 1, with the housing 3 removed.

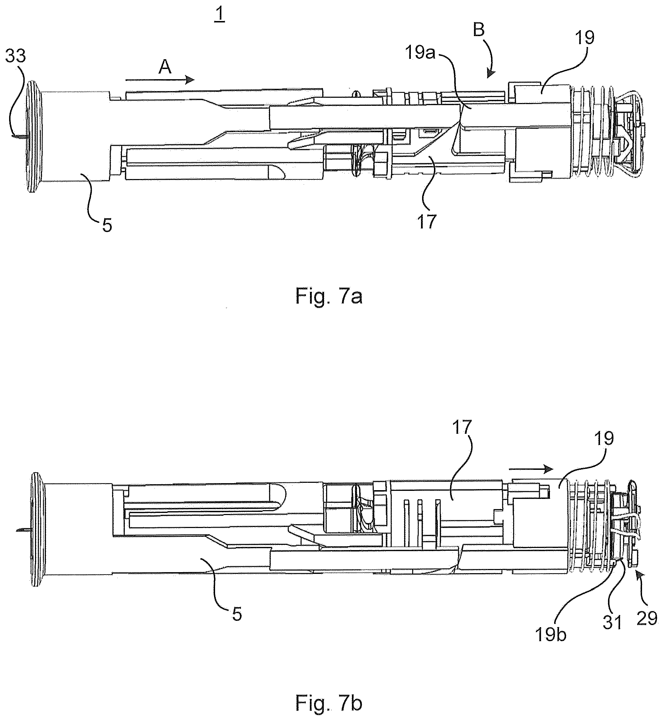

With reference to Figs 7a-9c, the medicament administration procedure utilising medicament delivery device 1 will now be described. The delivery member cover 5 is in this procedure pressed towards the injection site, causing the delivery member cover 5 to move proximally into the housing 3, from the extended position to the retracted position, i.e. from the first positon to the second position.

Fig. 7a shows a situation in which the delivery member cover 5 has been moved from the first position towards the second position relative to the movable sleeve 17, as shown by arrow A, to thereby expose the delivery member 33 which according to the present example is a needle. This causes a rotation of the movable sleeve 17, as shown by arrow B. The delivery member cover 5 has just been set in contact with the proximally extending leg 19a of the activation sleeve 19, and has moved the activation sleeve 19 slightly in the distal direction.

Fig. 7b shows a slight rotation of the entire medicament delivery device 1, to better visualise the effect of the distal movement of the activation sleeve 19.

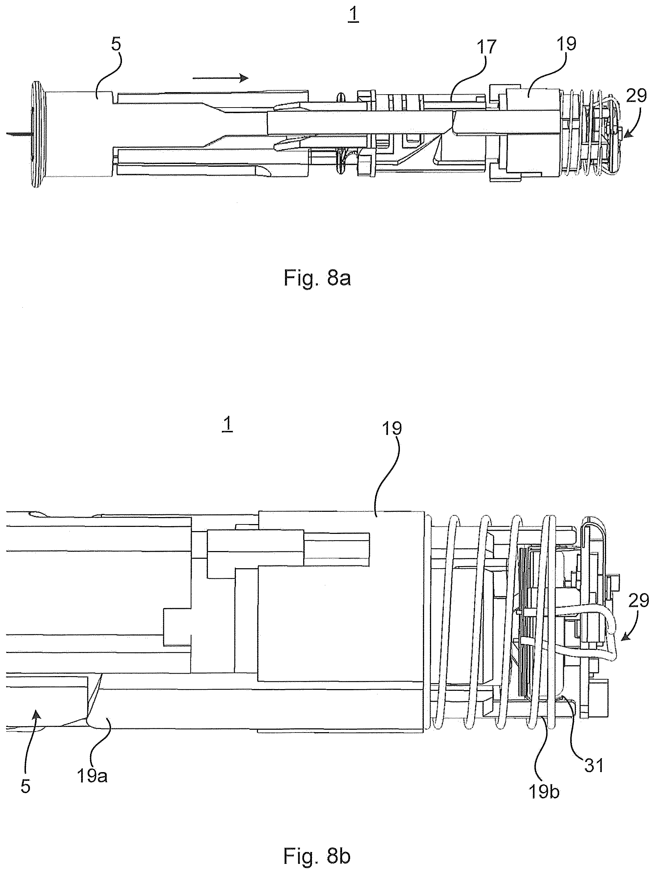

Fig. 8a depicts a stage of the medicament administration procedure when the delivery member cover 5 has been moved even further in the distal direction, essentially to the second position relative to the movable sleeve 17. The movable sleeve 17 has thus been further rotated, and essentially obtained its second rotational position.

Fig. 8b shows a close-up view of a distal end portion of the medicament delivery device 1 in the state shown in Fig. 8a, but with the medicament delivery device 1 having been slightly rotated. The distally extending arm 19b has moved past the switch 31 axially. The switch 31, which initially extended radially beyond the axial path of the distally extending arm 19b, has thus been pressed radially inwards by the distally extending arm 19b. The distally extending arm 19b hence contacts the switch 31, and presses it radially inwards. As long as the delivery member cover 5 is in the second position, or is pushed sufficiently in the distal direction, the switch 31 will maintain contact with the distally extending arm 19b and it will consequently be pressed radially inwards during this time. The activation sleeve 19 hence provides a first actuation of the switch 31.

The switch 31 may for example trigger the energy storage unit to power the processing circuitry, and/or it may cause the processing circuitry to register activation, the initiation of a medicament administration procedure, of the medicament delivery device 1.

The stroke, or stroke length, of the activation sleeve 19 is relatively long, while the stroke length of the switch is relatively short compared to the stroke length of the activation sleeve 19. Since the distally extending arm 19b is allowed to move axially past the switch 31 while actuating it, the mechanical tolerances between these cooperating members does not have to be that precise, which is highly advantageous in a manufacturing process.

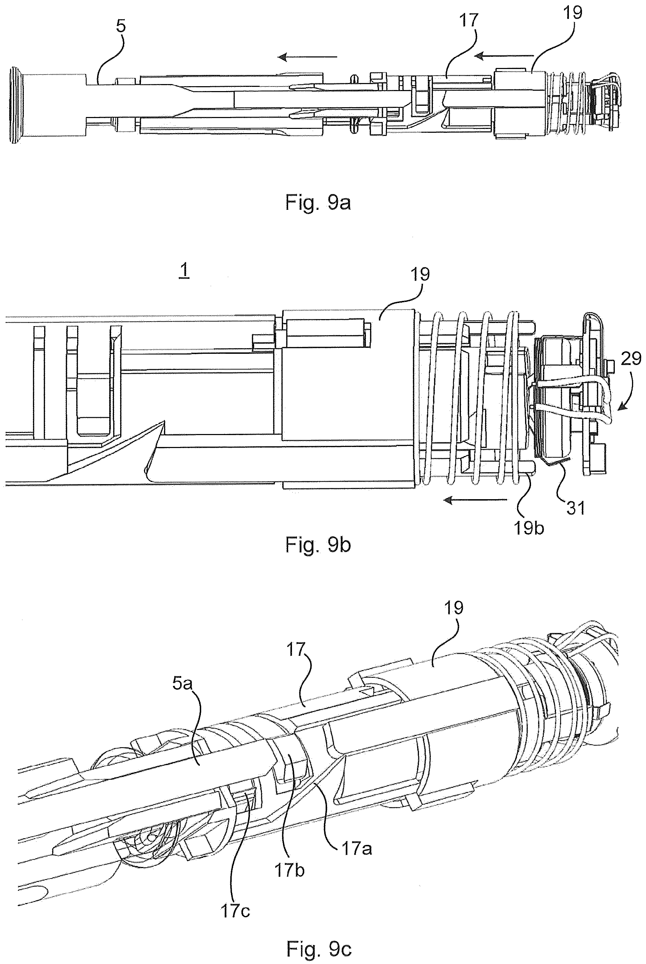

Fig. 9a shows the medicament delivery device 1 when the proximally biased delivery member cover 5 has returned from the second position to the first position. It can be noted that the movable sleeve 17 remains in the second rotational position. Due to the proximal biasing of the activation sleeve 19, the activation sleeve 19 is returned to its initial position, concurrently with the proximal movement of the delivery member cover 5.

Fig. 9b shows a close-up view of the distal end portion of the medicament delivery device 1, in the state shown in Fig. 9a but with the medicament delivery device 1 having been slightly rotated. As can be seen, the distally extending arm 19b has moved axially in the proximal direction, causing a release of contact with the switch 31. The activation sleeve 19 hence provides a second actuation of the switch 31, when the contact between the activation sleeve 19 and the switch 31 ceases. Since the delivery member cover 5 is moved back towards the first position when the medicament delivery device 1 is removed from the injection site, this second actuation also provides an indication that the medicament administration procedure is being finalised or completed.

The second actuation of the switch 31 may thus for example cause a registration of the completion of the medicament administration procedure by the electronics assembly 29. Additionally, the second actuation may for example also cause the processing circuitry to transmit the previously registered commencement of medicament administration procedure by means of the wireless transmitter and/or of the registration of completion of the medicament administration procedure. According to on example, the electronics assembly may be configured to shut down after a predetermined amount of time after the second actuation.

Fig. 9c shows the final state of the medicament delivery device 1, as shown in Figs 9a and 9b. Since the movable sleeve 17 has been rotated, and is in the second rotational position, the first tongue 17b is arranged in front of, i.e. axially aligned with and distally arranged from the first leg 5a of the delivery member cover 5. The delivery member cover 5 is thus prevented from being moved distally towards the second position once it has returned to the first position. Additionally, the radially outwards extending protrusion 17c is now arranged between the radially extending portion 17a and the first leg 5a of the delivery member cover 5, preventing the movable sleeve 17 from rotating back from the second rotational position to the first rotational position.

According to one example of the medicament delivery device 1, the tubular extension member 15 may be provided with radially flexible arms, and the medicament delivery device 1 may comprise a signal generating member, for example a U-shaped bracket provided with two longitudinally extending legs, configured to be received by the tubular extension member and the plunger rod 13 configured to be received between the legs of the U-shaped bracket. The movable sleeve 17 is configured to receive the tubular extension member 15, and the radially flexible arms are configured to be pressed radially inwards by the inner surface of the movable sleeve. The plunger rod 13, which has radial recesses configured to receive a respective one of the radially flexible arms therein, is thereby maintained in an initial axial position. The movable sleeve 17 may furthermore have an inner surface provided with recesses. When the movable sleeve 17 is being rotated, the radially flexible arms, which initially are pressed radially inwards by the inner surface of the movable sleeve, will be able to flex radially outwards, into the recesses of the movable sleeve 17. The plunger rod 13 is thereby released from its axially fixed position. The medicament delivery device 1 may also include a resilient member, for example a spring, configured to bias the U-shaped bracket in the distal direction, and a movable rod provided distally from and axially aligned with the U-shaped bracket. When the plunger rod 13 is released and moved in the proximal direction, the resilient member will cause the U-shaped bracket to be thrown backwards, i.e. in the distal direction towards the movable rod, which may be configured to actuate a sensor or switch different from the sensor 31. In this case, this audible click may be used as additional input concerning the stages of the medicament administration procedure. This audible click should appear prior to the second actuation of the switch 31, and it provides an indication that the medicament expulsion has been completed successfully. For this purpose, the electronics assembly may be configured to determine the time between the first actuation of the switch 31 and the detection of the audible click and/or the time between the detection of the audible click and the second actuation of the switch 31.

The inventive concept has mainly been described above with reference to a few examples. However, as is readily appreciated by a person skilled in the art, other embodiments than the ones disclosed above are equally possible within the scope of the inventive concept, as defined by the appended claims.

## Claims

1. A medicament delivery device (1) comprising: a housing (3), and an activation assembly (27) for a medicament delivery device (1), wherein the activation assembly (27) comprises:
a delivery member cover (5) provided with legs (5a, 5b) and configured to be biased in a proximal direction,
a movable sleeve (17) configured to be received between the legs (5a, 5b), and
wherein the delivery member cover (5) is configured to be moved in an axial direction between a first position relative to the movable sleeve (17) and a second position relative to the movable sleeve (17),
wherein the movable sleeve (17) is rotatable relative to the delivery member cover (5), and linear movement of the delivery member cover (5) from the first position to the second position causes rotation of the movable sleeve (17), wherein the first movable sleeve position is a first rotational position and the second movable sleeve position is a second rotational position,
wherein the delivery member cover (5) has a distal end surface (5c), defining a respective end of the first leg (5a) and the second leg (5b), configured to cooperate with a radially extending portion (17a) of the movable sleeve (17), forming a cam surface, wherein cooperation between the distal end surface (5c) and the radially extending surface (17a) causes the rotation of the movable sleeve (17),
**characterized in that** the activation assembly (27) further comprises an activation sleeve (19) configured to be biased in the proximal direction,
wherein the delivery member cover (5) is configured to axially displace the activation sleeve (19) in a distal direction when the delivery member cover (5) is moved towards the second position, wherein the activation sleeve (19) has proximally extending arms (19a) configured to be axially aligned with and to cooperate with a respective leg (5a, 5b) of the delivery member cover (5), the delivery member cover (5) thereby pushing the activation sleeve (19) when moved from the first position to the second position causing the axial displacement of the activation sleeve (19), the activation sleeve (19) being configured to thereby provide a first actuation of a sensor (31) of an electronics assembly (29) of the medicament delivery device (1),
wherein the activation sleeve (19) has a distally extending arm (19b) configured to actuate the sensor (31);
wherein the activation sleeve (19) is configured to move in the proximal direction when the delivery member cover (5) returns from the second position to the first position, thereby providing a second actuation of the sensor (31), and wherein the movable sleeve (17) is configured to prevent the delivery member cover (5) from moving from the first position to the second position when the delivery member cover (5) has returned from the second position to the first position.

2. The medicament delivery device (1) as claimed in claim 1, wherein the movable sleeve (17) has a tangentially extending radially flexible first tongue (17b) configured to allow the delivery member cover (5) to move over the first tongue (17b) towards the second position when the movable sleeve (17) is in the first rotational position and to prevent the delivery member cover to move from the first position to the second position when the movable sleeve (17) is in the second rotational position and the delivery member cover (5) has returned to the first position.

3. The medicament delivery device (1) as claimed in claim 2, wherein the first tongue (17b) has an increasing thickness in the tangential direction, whereby the first tongue (17b) is configured to enable the delivery member cover (5) to move over the first tongue (17b), in the axial direction, from the first position towards the second position, when the movable sleeve (17) is in the first rotational position.

4. The medicament delivery device (1) as claimed in any of claims 1-3, wherein the movable sleeve (17) has a radially outwards extending protrusion (17c) configured to cooperate with the delivery member cover (5) to prevent rotation of the movable sleeve (17) from the second rotational position to the first rotational position.

5. The medicament delivery device (1) as claimed in claim 4, wherein the movable sleeve (17) has a tangentially extending radially flexible second tongue (17d), wherein the radially outwards extending protrusion (17c) is provided on the second tongue (17d).

6. The medicament delivery device (1) as claimed in any of the preceding claims, wherein the electronics assembly (29) including an energy storage unit and processing circuitry configured to register activation of a medicament administration procedure.

7. The medicament delivery device (1) as claimed in claim 6, wherein the sensor (31) is in the form of a switch, and the activation sleeve (19) is configured to provide the first actuation of the switch.

8. The medicament delivery device (1) as claimed in claim 7, wherein the switch extends radially outwards and is radially flexible, wherein the activation sleeve (19) is configured to provide the first actuation of the switch by moving distally, over the switch, thereby pressing the switch radially inwards and to provide the second actuation of the switch by moving proximally, away from the switch, thereby releasing the switch from being pressed radially inwards.

9. The activation assembly (27) as claimed in any of the preceding claims, wherein the movable sleeve (17) is configured to be arranged proximally relative to the activation sleeve (19).

10. The medicament delivery device (1) as claimed in claim 1, comprising a plunger rod (13) configured to move axially inside the housing (3) from an initial position to an end position, and an end click member configured to be released and thrown in the distal direction when the plunger rod (13) has travelled a majority of the distance from the initial position to the end position, and an end click sensor configured to detect impact of the tend click member upon being released, causing the electronics assembly (29) to register a completion of medicament expulsion.

## Patentansprüche

1. Medikamentenabgabevorrichtung (1), umfassend: ein Gehäuse (3) und eine Aktivierungsanordnung (27) für eine Medikamentenabgabevorrichtung (1), wobei die Aktivierungsanordnung (27) umfasst:
eine Abgabeelementabdeckung (5), die mit Schenkeln (5a, 5b) versehen ist und dazu konfiguriert ist, in eine proximale Richtung vorgespannt zu werden,
eine bewegliche Hülse (17), die dazu konfiguriert ist, zwischen den Schenkeln (5a, 5b) aufgenommen zu werden, und
wobei die Abgabeelementabdeckung (5) dazu konfiguriert ist, in einer axialen Richtung zwischen einer ersten Position bezüglich der beweglichen Hülse (17) und einer zweiten Position bezüglich der beweglichen Hülse (17) bewegt zu werden,
wobei die bewegliche Hülse (17) bezüglich der Abgabeelementabdeckung (5) drehbar ist und eine lineare Bewegung der Abgabeelementabdeckung (5) aus der ersten Position in die zweite Position eine Drehung der beweglichen Hülse (17) bewirkt, wobei die erste Position der beweglichen Hülse eine erste Drehposition ist und die zweite Position der beweglichen Hülse eine zweite Drehposition ist,
wobei die Abgabeelementabdeckung (5) eine distale Endfläche (5c) aufweist, die ein jeweiliges Ende des ersten Schenkels (5a) und des zweiten Schenkels (5b) definiert, das dazu konfiguriert ist, mit einem sich radial erstreckenden Abschnitt (17a) der beweglichen Hülse (17) zusammenzuwirken, der eine Nockenoberfläche bildet, wobei ein Zusammenwirken zwischen der distalen Endfläche (5c) und der sich radial erstreckenden Oberfläche (17a) die Drehung der beweglichen Hülse (17) bewirkt,
**dadurch gekennzeichnet, dass** die Aktivierungsanordnung (27) ferner eine Aktivierungshülse (19) umfasst, die dazu konfiguriert ist, in die proximale Richtung vorgespannt zu werden,
wobei die Abgabeelementabdeckung (5) dazu konfiguriert ist, die Aktivierungshülse (19) in einer distalen Richtung axial zu verschieben, wenn die Abgabeelementabdeckung (5) in Richtung der zweiten Position bewegt wird, wobei die Aktivierungshülse (19) sich proximal erstreckende Arme (19a) aufweist, die dazu konfiguriert sind, mit einem jeweiligen Schenkel (5a, 5b) der Abgabeelementabdeckung (5) axial ausgerichtet zu sein und zusammenzuwirken, wobei die Abgabeelementabdeckung (5) dadurch die Aktivierungshülse (19) drückt, wenn sie aus der ersten Position in die zweite Position bewegt wird, was die axiale Verschiebung der Aktivierungshülse (19) bewirkt, wobei die Aktivierungshülse (19) dazu konfiguriert ist, dadurch eine erste Betätigung eines Sensors (31) einer Elektronikbaugruppe (29) der Medikamentenabgabevorrichtung (1) bereitzustellen,
wobei die Aktivierungshülse (19) einen sich distal erstreckenden Arm (19b) aufweist, der dazu konfiguriert ist, den Sensor (31) zu betätigen;
wobei die Aktivierungshülse (19) dazu konfiguriert ist, sich in die proximale Richtung zu bewegen, wenn die Abgabeelementabdeckung (5) aus der zweiten Position in die erste Position zurückkehrt, wodurch eine zweite Betätigung des Sensors (31) bereitgestellt wird und wobei die bewegliche Hülse (17) dazu konfiguriert ist, zu verhindern, dass sich die Abgabeelementabdeckung (5) aus der ersten Position in die zweite Position bewegt, wenn die Abgabeelementabdeckung (5) aus der zweiten Position in die erste Position zurückgekehrt ist.

2. Medikamentenabgabevorrichtung (1) nach Anspruch 1, wobei die bewegliche Hülse (17) eine sich tangential erstreckende, radial flexible erste Zunge (17b) aufweist, die dazu konfiguriert ist, es der Abgabeelementabdeckung (5) zu ermöglichen, sich über die erste Zunge (17b) in Richtung der zweiten Position zu bewegen, wenn die bewegliche Hülse (17) in der ersten Drehposition ist, und zu verhindern, dass sich die Abgabeelementabdeckung aus der ersten Position in die zweite Position bewegt, wenn die bewegliche Hülse (17) in der zweiten Drehposition ist und die Abgabeelementabdeckung (5) in die erste Position zurückgekehrt ist.

3. Medikamentenabgabevorrichtung (1) nach Anspruch 2, wobei die erste Zunge (17b) eine zunehmende Dicke in der tangentialen Richtung aufweist, wobei die erste Zunge (17b) dazu konfiguriert ist, es der Abgabeelementabdeckung (5) zu erlauben, sich über die erste Zunge (17b) in der axialen Richtung aus der ersten Position in Richtung der zweiten Position zu bewegen, wenn die bewegliche Hülse (17) in der ersten Drehposition ist.

4. Medikamentenabgabevorrichtung (1) nach einem der Ansprüche 1-3, wobei die bewegliche Hülse (17) einen sich radial nach außen erstreckenden Vorsprung (17c) aufweist, der dazu konfiguriert ist, mit der Abgabeelementabdeckung (5) zusammenzuwirken, um eine Drehung der beweglichen Hülse (17) aus der zweiten Drehposition in die erste Drehposition zu verhindern.

5. Medikamentenabgabevorrichtung (1) nach Anspruch 4, wobei die bewegliche Hülse (17) eine sich tangential erstreckende, radial flexible zweite Zunge (17d) aufweist, wobei der sich radial nach außen erstreckende Vorsprung (17c) an der zweiten Zunge (17d) bereitgestellt ist.

6. Medikamentenabgabevorrichtung (1) nach einem der vorstehenden Ansprüche, wobei die Elektronikbaugruppe (29) eine Energiespeichereinheit und einen Verarbeitungsschaltkreis einschließt, der dazu konfiguriert ist, eine Aktivierung eines Medikamentenverabreichungsvorgangs aufzuzeichnen.

7. Medikamentenabgabevorrichtung (1) nach Anspruch 6, wobei der Sensor (31) in der Form eines Schalters ist und die Aktivierungshülse (19) dazu konfiguriert ist, die erste Betätigung des Schalters bereitzustellen.

8. Medikamentenabgabevorrichtung (1) nach Anspruch 7, wobei sich der Schalter radial nach außen erstreckt und radial flexibel ist, wobei die Aktivierungshülse (19) dazu konfiguriert ist, die erste Betätigung des Schalters durch distales Bewegen über den Schalter bereitzustellen, wodurch der Schalter radial nach innen gedrückt wird, und die zweite Betätigung des Schalters durch proximales Bewegen weg von dem Schalter bereitzustellen, wodurch der radial nach innen gedrückte Schalter gelöst wird.

9. Aktivierungsanordnung (27) nach einem der vorstehenden Ansprüche, wobei die bewegliche Hülse (17) dazu konfiguriert ist, proximal bezüglich der Aktivierungshülse (19) angeordnet zu werden.

10. Medikamentenabgabevorrichtung (1) nach Anspruch 1, umfassend eine Kolbenstange (13), die dazu konfiguriert ist, sich axial innerhalb des Gehäuses (3) aus einer Ausgangsposition in eine Endposition zu bewegen, und ein Endklickelement, das dazu konfiguriert ist, gelöst und in die distale Richtung geworfen zu werden, wenn die Kolbenstange (13) einen Großteil des Abstands aus der Ausgangsposition in die Endposition gewandert ist, und einen Endklicksensor, der dazu konfiguriert ist, ein Auftreffen des Endklickelements beim Lösen zu erfassen, wodurch bewirkt wird, dass die Elektronikbaugruppe (29) einen Abschluss des Medikamentenausstoßes aufzeichnet.

## Revendications

1. Dispositif de distribution de médicament (1) comprenant : un logement (3), et un ensemble d'activation (27) pour un dispositif de distribution de médicament (1), dans lequel l'ensemble d'activation (27) comprend :
un couvercle d'élément de distribution (5) pourvu de pattes (5a, 5b) et configuré pour être sollicité dans une direction proximale,
un manchon mobile (17) configuré pour être reçu entre les pattes (5a, 5b), et
dans lequel le couvercle d'élément de distribution (5) est configuré pour être déplacé dans une direction axiale entre une première position par rapport au manchon mobile (17) et une deuxième position par rapport au manchon mobile (17),
dans lequel le manchon mobile (17) est rotatif par rapport au couvercle d'élément de distribution (5), et un mouvement linéaire du couvercle d'élément de distribution (5) de la première position à la deuxième position amène une rotation du manchon mobile (17), dans lequel la première position de manchon mobile est une première position de rotation et la deuxième position de manchon mobile est une deuxième position de rotation,
dans lequel le couvercle d'élément de distribution (5) a une surface d'extrémité distale (5c), définissant une extrémité respective de la première patte (5a) et de la deuxième patte (5b), configurée pour coopérer avec une partie s'étendant radialement (17a) du manchon mobile (17), formant une surface de came, dans lequel une coopération entre la surface d'extrémité distale (5c) et la surface s'étendant radialement (17a) amène la rotation du manchon mobile (17),
**caractérisé en ce que** l'ensemble d'activation (27) comprend en outre un manchon d'activation (19) configuré pour être sollicité dans la direction proximale,
dans lequel le couvercle d'élément de distribution (5) est configuré pour déplacer axialement le manchon d'activation (19) dans une direction distale lorsque le couvercle d'élément de distribution (5) est déplacé en direction de la deuxième position, dans lequel le manchon d'activation (19) a des bras s'étendant proximalement (19a) configurés pour être alignés axialement avec et pour coopérer avec une patte respective (5a, 5b) du couvercle d'élément de distribution (5), le couvercle d'élément de distribution (5) poussant de ce fait le manchon d'activation (19) lorsqu'il est déplacé de la première position à la deuxième position amenant le déplacement axial du manchon d'activation (19), le manchon d'activation (19) étant configuré pour fournir de ce fait un premier actionnement d'un capteur (31) d'un ensemble électronique (29) du dispositif de distribution de médicament (1),
dans lequel le manchon d'activation (19) a une un bras s'étendant distalement (19b) configuré pour actionner le capteur (31) ;
dans lequel le manchon d'activation (19) est configuré pour se déplacer dans la direction proximale lorsque le couvercle d'élément de distribution (5) retourne de la deuxième position à la première position, en fournissant de ce fait un deuxième actionnement du capteur (31), et dans lequel le manchon mobile (17) est configuré pour empêcher le couvercle d'élément de distribution (5) de se déplacer de la première position à la deuxième position lorsque le couvercle d'élément de distribution (5) est retourné de la deuxième position à la première position.

2. Dispositif de distribution de médicament (1) tel que revendiqué dans la revendication 1, dans lequel le manchon mobile (17) a une première languette radialement flexible s'étendant tangentiellement (17b) configurée pour permettre au couvercle d'élément de distribution (5) de se déplacer par-dessus la première languette (17b) en direction de la deuxième position lorsque le manchon mobile (17) est dans la première position de rotation et pour empêcher le couvercle d'élément de distribution de se déplacer de la première position à la deuxième position lorsque le manchon mobile (17) est dans la deuxième position de rotation et que le couvercle d'élément de distribution (5) est retourné à la première position.

3. Dispositif de distribution de médicament (1) tel que revendiqué dans la revendication 2, dans lequel la première languette (17b) a une épaisseur croissante dans la direction tangentielle, moyennant quoi la première languette (17b) est configurée pour permettre au couvercle d'élément de distribution (5) de se déplacer par-dessus la première languette (17b), dans la direction axiale, de la première position en direction de la deuxième position, lorsque le manchon mobile (17) est dans la première position de rotation.

4. Dispositif de distribution de médicament (1) tel que revendiqué dans l'une quelconque des revendications 1 à 3, dans lequel le manchon mobile (17) a une partie saillante s'étendant radialement vers l'extérieur (17c) configurée pour coopérer avec le couvercle d'élément de distribution (5) pour empêcher une rotation du manchon mobile (17) de la deuxième position de rotation à la première position de rotation.

5. Dispositif de distribution de médicament (1) tel que revendiqué dans la revendication 4, dans lequel le manchon mobile (17) a une deuxième languette radialement flexible s'étendant tangentiellement (17d), dans lequel la partie saillante s'étendant radialement vers l'extérieur (17c) est fournie sur la deuxième languette (17d).

6. Dispositif de distribution de médicament (1) tel que revendiqué dans l'une quelconque des revendications précédentes, dans lequel l'ensemble électronique (29) inclut une unité de stockage d'énergie et un circuit de traitement configuré pour enregistrer l'activation d'une procédure d'administration de médicament.

7. Dispositif de distribution de médicament (1) tel que revendiqué dans la revendication 6, dans lequel le capteur (31) est sous la forme d'un commutateur, et le manchon d'activation (19) est configuré pour fournir le premier actionnement du commutateur.

8. Dispositif de distribution de médicament (1) tel que revendiqué dans la revendication 7, dans lequel le commutateur s'étend radialement vers l'extérieur et est radialement flexible, dans lequel le manchon d'activation (19) est configuré pour fournir le premier actionnement du commutateur en se déplaçant distalement, par-dessus le commutateur, en pressant de ce fait le commutateur radialement vers l'intérieur et pour fournir le deuxième actionnement du commutateur en se déplaçant proximalement, à l'écart du commutateur, en libérant de ce fait le commutateur son état pressé radialement vers l'intérieur.

9. Ensemble d'activation (27) tel que revendiqué dans l'une quelconque des revendications précédentes, dans lequel le manchon mobile (17) est configuré pour être agencé proximalement par rapport au manchon d'activation (19).

10. Dispositif de distribution de médicament (1) tel que revendiqué dans la revendication 1, comprenant une tige de piston (13) configurée pour se déplacer axialement à l'intérieur du logement (3) d'une position initiale à une position finale, et un élément à déclic d'extrémité configuré pour être libéré et lancé dans la direction distale lorsque la tige de piston (13) a parcouru une majorité de la distance allant de la position initiale à la position finale, et un capteur de déclic d'extrémité configuré pour détecter un impact de l'élément à déclic d'extrémité lorsqu'il est libéré, amenant l'ensemble électronique (29) à enregistrer un achèvement d'expulsion de médicament.
